# EUROPEAN PATENT APPLICATION

(11) **EP 4 386 766 A1**
(43) Date of publication of application: **19.06.2024**
(21) Application number: 22214349.7
(22) Date of filing: 16.12.2022
(51) Int. Cl.: G16C 20/30

(54) **METHOD AND SYSTEM FOR PREDICTING A STABILITY VALUE FOR A DETERMINED FRAGRANCE IN A DETERMINED FRAGRANCE BASE**

(71) Applicant: Firmenich SA, 1242 Satigny (CH)
(72) Inventor: CHICHESTER, Christine, 1242 Satigny (CH); SHIFERAW, Addisalem, 1242 Satigny (CH); TVERDOKHLEBOVA, Elena, 1015 Lausanne (CH)
(74) Representative: dsm-firmenich IP

(57) **Abstract**

The method (100) for predicting a stability value for a determined fragrance in a determined fragrance base, comprises:
- a step (105) of inputting, upon a computer interface, at least two fragrance ingredient digital identifiers, representative of material fragrance ingredients, the resulting input corresponding to a composition of fragrance ingredients,
- a step (110) of inputting, upon a computer interface, a fragrance base digital identifier, representative of a material fragrance base,
- a step (115) of determination, by a computing device, of a stability value for the input composition in the input fragrance base, as a function of the input composition and the input fragrance base and
- a step (120) of providing, upon a computer interface, a value representative of the determined stability value.

## Description

### TECHNICAL FIELD OF THE INVENTION

The present invention aims at a method for predicting a stability value for a determined fragrance in a determined fragrance base and to a system for predicting a stability value for a determined fragrance in a determined fragrance base.

It applies, in particular, to the industry of fragrance design and production.

### BACKGROUND OF THE INVENTION

The approaches described in this section are approaches that could be pursued, but not necessarily approaches that have been previously conceived or pursued. Therefore, unless otherwise indicated, it should not be assumed that any of the approaches described in this section qualify as prior art merely by virtue of their inclusion in this section.

In the field of fragrance design and production, one of the key industrial challenges is to provide fragrances that contribute to the commercial success of a product. Such products include, for example, haircare products and fine perfumery. Such products, while consumable, are typically consumed over significant lengths of time. A shampoo may be consumed over several weeks and a perfume may be consumed over several months, for instance. Therefore, one of the key metrics when designing a fragranced product is called stability, or robustness, and corresponds to, for example, the colorimetric, solubility or olfactive stability of the product over time. For example, a toothpaste must remain a certain color to satisfy its user and a perfume must not change in smell as time passes.

Such stability is impacted by the selection of particular fragrance ingredients as well as the selection of a product base, which corresponds, conceptually, to the chemical substrate which is scented by the interaction with the composition of fragrance ingredients. A base may correspond, for example, to the chemical formula forming the active part of a toothpaste.

In traditional approaches, prior to mass industrialization of a product, prototypes of the product are assembled and analyzed to provide a quantitative assessment of, for example, the colorimetric, solubility or olfactive stability of the product.

Such approaches are costly, both in terms of time required as well as corresponding financial expenditure. Furthermore, such approaches may provide incoherent results due to the limited sample size. The outcome of such trial and error based approaches is that significant product base or fragrance composition reformulations must be performed. Typically, such reformulations may be performed independently upon the base and fragrance compositions, without clear stability impact knowledge leading into said reformulations.

For these reasons, current approaches are unsatisfactory to reliably proactively evaluate the stability of a product and thus limit the product development quality and timeliness.

### SUMMARY OF THE INVENTION

The present invention aims at addressing all or part of these drawbacks.

All embodiments disclosed and claimed herein are directed to a computer-implemented programmed processes that interact with digital data to provide a practical application of computing technology to the problem of providing accurate stability predictions that can be used to design and produce stable scented products (chemical compositions). The disclosure is not intended to encompass techniques for organizing human activity, for performing mental processes, or for performing a mathematical concept, and any interpretation of the claims to encompass such techniques would be unreasonable based upon the disclosure as a whole.

According to a first aspect, the present invention aims at a method for predicting a stability value for a determined fragrance in a determined fragrance base, comprising:
- a step of inputting, upon a computer interface, at least two fragrance ingredient digital identifiers, representative of material fragrance ingredients, the resulting input corresponding to a composition of fragrance ingredients,
- a step of inputting, upon a computer interface, a fragrance base digital identifier, representative of a material fragrance base,
- a step of determination, by a computing device, of a stability value for the input composition in the input fragrance base, as a function of the input composition and the input fragrance base and
- a step of providing, upon a computer interface, a value representative of the determined stability value.

Such provisions allow for the prediction of the stability of a product, thus improving the speed and capacity to design and produce said products.

In optional embodiments, the method object of the present invention comprises a step of inputting, upon a computer interface, an fragrance application digital identifier, representative of the use the input fragrance and the input base.

Such embodiments further improve the quality of the prediction by modelling the impact of the application, or intended use, of the product. Indeed, depending upon the application, the environmental impact (in terms of conditioning medium or of luminosity of a typical storage of the product) impacts the stability of the product.

In optional embodiments, the step of determination comprises a step of operating a trained classifier machine learning device, in which input composition and the input fragrance base are used as input of the classifier machine learning model and in which the output is a stability class identifier, representative of a stability of the input composition in the input fragrance base.

Such embodiments allow for the determination of the stability of a product based upon fragrance composition, base and optionally application values.

In optional embodiments, the method object of the present invention comprises upstream of the step of operating a trained classifier machine learning device:
- providing to a classifier machine learning device a set of exemplar data, comprising:
   - composition digital identifiers, each composition digital identifier being associated with fragrance ingredient digital identifiers,
   - for at least part of the exemplar composition digital identifiers, a fragrance base digital identifier, and
   - for at least one couple formed of the exemplar composition and base digital identifiers, a value representative of stability of the exemplar composition,
- operating the classifier machine learning device based upon the set of exemplar data and
- obtaining the trained classifier machine learning device.

Such embodiments allow for the determination of the stability of a product based upon fragrance composition, base and optionally application values.

In optional embodiments, the exemplar data further comprises, for at least one couple formed of exemplar composition and base digital identifiers, a value representative of duration from assembly of said couple.

Such embodiments allow for the determination of the stability of a product which includes the modelling of the impact of duration since production of a product.

In optional embodiments, the exemplar data further comprises for at least one couple formed of exemplar composition and base digital identifiers, a value representative of the concentration of the composition within the base of said couple.

Such embodiments allow for the determination of the stability of a product which includes the modelling of the impact of the concentration of a fragrance composition in a base of a product.

In optional embodiments, the exemplar data further comprises for at least one couple formed of exemplar composition and base digital identifiers, a value representative of an application for said couple.

Such embodiments allow for the determination of the stability of a product which includes the modelling of the impact of the application of a product.

In optional embodiments, the trained classifier machine learning model is a classifier gradient boosting decision tree device.

In optional embodiments, the method object of the present invention comprises, downstream of the step of operating the trained gradient boosting decision tree device, a step of determining, by a computing device, a numerical value representative of a stability impact for at least one input fragrance ingredient digital identifier and a step of providing, upon a computer interface, the determined stability impact numerical value.

Such embodiments allow for the isolation of the most or least performing fragrance ingredients in terms of stability.

In optional embodiments, the method object of the present invention comprises, downstream of the step of determining a numerical value representative of a stability impact, a step of providing at least one alternative fragrance ingredient digital identifier for at least one fragrance ingredient digital identifier input to form the composition as function of the numerical value representative of a stability impacts of the said input and of said alternative fragrance ingredient digital identifiers.

Such embodiments allow for the dynamic reformulation of a fragrance composition in order to increase the stability of the composition.

In optional embodiments, the step of providing at least one alternative fragrance ingredient digital identifier is configured to further provide at least one value representative of a concentration of at least one said alternative fragrance ingredient digital identifier.

In optional embodiments, the step of providing at least one alternative fragrance ingredient digital identifier is configured to further provide a minimum and/or a maximum value representative of a concentration of at least one said alternative fragrance ingredient digital identifier.

In optional embodiments, the method object of the present invention comprises a step of assembling the input composition in the input fragrance base.

Such provisions allow for the materialization of the composition and base into a final product.

According to a second aspect, the present invention aims at a system for predicting a stability value for a determined fragrance in a determined fragrance base, comprising:
- one or more computer systems comprising one or more hardware processors and storage media; and
- instructions stored in the storage media and which, when executed by the computing system, cause the computing system to perform:
   - a step of inputting, upon a computer interface, at least two fragrance ingredient digital identifiers, representative of material fragrance ingredients, the resulting input corresponding to a composition of fragrance ingredients,
   - a step of inputting, upon a computer interface, a fragrance base digital identifier, representative of a material fragrance base,
- a step of determination, by a computing device, of a stability value for the input composition in the input fragrance base, as a function of the input composition and the input fragrance base and
- a step of providing, upon a computer interface, a value representative of the determined stability value.

Such provisions provide similar benefits to those of the first aspect of the present invention.

### BRIEF DESCRIPTION OF THE DRAWINGS

Other advantages, purposes and particular characteristics of the invention shall be apparent from the following non-exhaustive description of at least one particular embodiment of the method or system object of the present invention, in relation to the drawings annexed hereto, in which:
- Figure 1 shows, schematically, a first particular succession of steps of the method object of the present invention,
- Figure 2 shows, schematically, a particular embodiment of the system object of the present invention and
- Figure 3 shows, schematically, a second particular succession of steps of the method object of the present invention.

### DETAILED DESCRIPTION OF THE INVENTION

This description is not exhaustive, as each feature of one embodiment may be combined with any other feature of any other embodiment in an advantageous manner.

Various inventive concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should be noted at this point that the figures are not to scale.

As used herein, the terms 'ingredient' designates a perfuming ingredient, a flavor ingredient, a perfumery carrier, a flavor carrier, a perfumery adjuvant, a flavor adjuvant, a perfumery modulator, flavor modulator. Preferably, such a fragrance or fragrant chemical compound is volatile. Such an ingredient may be a natural ingredient. An ingredient is intended in the sense of an ingredient selected for the contribution of said ingredient to a designed flavor or fragrance. Such ingredients are sometimes defined by a CAS (for "Chemical Abstracts Service") number.

By 'perfuming ingredient' it is meant here a compound, which is used in a perfuming preparation or a composition to impart a hedonic effect. In other words, such an ingredient, to be considered as being a perfuming one, must be recognized by a person skilled in the art as being able to impart or modify in a positive or pleasant way the odor of a composition, and not just as having an odor.

The nature and type of the perfuming ingredients do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Said perfuming ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said perfuming ingredients may also be compounds known to release in a controlled manner various types of perfuming ingredients also known as properfume or profragrance.

By 'perfumery carrier' it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, benzyl benzoate, benzyl alcohol, 2-(2-ethoxyethoxy)-1-ethano, tri-ethyl citrate or mixtures thereof, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. The use of solid carriers is of current use in the art and a person skilled in the art knows how to reach the desired effect. However, by way of non-limiting examples of solid carriers, one may cite absorbing gums or polymers or inorganic materials, such as porous polymers, cyclodextrins, wood based materials, organic or inorganic gels, clays, gypsum talc or zeolites.

As other non-limiting examples of solid carriers, one may cite encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs-und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture threof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycoluryl, melamine, methylol melamine, methylated methylol melamine, guanazole and the like, as well as mixtures thereof. Alternatively, one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Others resins one are the ones produced by the polycondensation of an a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine-based resins with aldehydes includes represented by articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinencypertinence, which disclose suitable uses of such microcapsules, are represented for example by the article of K. Bruyninckx and M. Dusselier, ACS Sustainable Chemistry & Engineering, 2019, vol. 7, pages 8041-8054H.Y.Lee et al. Journal of Microencapsulation, 2002, vol. 19, pages 559-569, international patent publication WO 01/41915 or yet the article of S. Bône et al. Chimia, 2011, vol. 65, pages 177-181.

By 'perfumery adjuvant', it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof.

By 'perfumery modulator', it is understood here an agent having the capacity to affect the manner in which the odour, and in particular the evaporation rate and intensity, of the compositions incorporating said modulator can be perceived by an observer or user thereof, over time, as compared to the same perception in the absence of the modulator. Perfumery modulators are also known as fixative. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate; panthenol ethyl ether, DL-panthenol, N-hexadecyl n-nonanoate, noctadecyl n-nonanoate, a profragrance, cyclodextrin, an encapsulation, and a combination thereof.

By 'flavoring ingredient' it is meant here a compound, which is used in flavoring preparations or compositions to impart a hedonic effect. In other words, such an ingredient, to be considered as being a flavoring one, must be recognized by a person skilled in the art as being able to impart or modify in a positive or pleasant way the taste of a composition, and not just as having a taste. The nature and type of the flavoring ingredients present in the composition do not warrant a more detailed description here, the skilled person being able to select them on the basis of its general knowledge and according to intended use or application and the desired organoleptic effect. In general terms, these flavoring ingredients belong to chemical classes as varied as alcohols, aldehydes, ketones, esters, ethers, acetates, nitriles, terpenoids, nitrogenous or sulphurous heterocyclic compounds and essential oils, and said flavoring ingredients can be of natural or synthetic origin. Many of these ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of flavor. It is also understood that said co-ingredients may also be compounds known to release in a controlled manner various types of flavoring compounds, also called pro-flavor.

The term 'flavor carrier' designates a material which is substantially neutral from a flavor point of view, insofar as it does not significantly alter the organoleptic properties of flavoring ingredients. The carrier may be a liquid or a solid.

Suitable liquid carriers include, for instance, an emulsifying system, i.e. a solvent and a surfactant system, or a solvent commonly used in flavors. A detailed description of the nature and type of solvents commonly used in flavor cannot be exhaustive. Suitable solvents used in flavor include, for instance, propylene glycol, triacetine, caprylic/capric triglyceride (neobee^{®}), triethyl citrate, benzylic alcohol, ethanol, isopropanol, citrus terpenes, vegetable oils such as Linseed oil, sunflower oil or coconut oil, glycerol.

Suitable solid carriers include, for instance, absorbing gums or polymers, or even encapsulating materials. Examples of such materials may comprise wall-forming and plasticizing materials, such as mono, di- or polysaccharides, natural or modified starches, hydrocolloids, cellulose derivatives, polyvinyl acetates, polyvinylalcohols, xanthan gum, arabic gum, acacia gum or yet the materials cited in reference texts such as H. Scherz, Hydrokolloid: Stabilisatoren, Dickungs-und Geliermittel in Lebensmitteln, Band 2 der Schriftenreihe Lebensmittelchemie, Lebensmittelqualität, Behr's VerlagGmbH & Co., Hamburg, 1996. Encapsulation is a well-known process to a person skilled in the art, and may be performed, for instance, using techniques such as spray-drying, agglomeration, extrusion, coating, plating, coacervation and the like.

By 'flavor adjuvant', it is meant here an ingredient capable of imparting additional added benefit such as a color (e.g. caramel), chemical stability, and so on. A detailed description of the nature and type of adjuvant commonly used in flavoring compositions cannot be exhaustive. Nevertheless, such adjuvants are well known to a person skilled in the art who will be able to select them on the basis of its general knowledge and according to intended use or application. One may cite as specific non-limiting examples the following: viscosity agents (e.g. emulsifier, thickeners, gelling and/or rheology modifiers, e.g. pectin or agar gum), stabilizing agents (e.g. antioxidant, heat/light and or buffers agents e.g. citric acid), coloring agents (e.g. natural or synthetic or natural extract imparting color), preservatives (e.g. antibacterial or antimicrobial or antifungal agents, e.g. benzoic acid), vitamins and mixtures thereof.

By 'flavor modulator', it is meant here an ingredient capable to enhance sweetness, to block bitterness, to enhance umami, to reduce sourness or licorice taste, to enhance saltiness, to enhance a cooling effect, or any combinations of the foregoing. Flavor modulators are also called trigeminal sensates.

The terms "composition" or formula designates a liquid, solid or gaseous assembly of at least one volatile ingredient.

As used herein, a "fragrance" refers to the olfactory perception resulting from the sum of odorant receptor(s) activation, enhancement, and inhibition (when present) by at least one volatile ingredient. Accordingly, by way of illustration and by no means intending to limit the scope of the present disclosure, a "fragrant" results from the olfactory perception arising from the sum of a first volatile ingredient that activates an odorant receptor associated with a coconut tonality, a second volatile ingredient that activates an odorant receptor associated with a celery tonality, and a third volatile ingredient that inhibits an odorant receptor associated with a hay tonality.

As used herein, the terms "digital identifier" refer to any computerized representation, such as one used in a computer database, representing a physical object, such as a flavoring ingredient. A digital identifier may refer to a label representative of the name, chemical structure or internal reference of the flavoring ingredient.

In the present description, the term 'materialized' is intended as existing outside of the digital environment of the present invention. 'Materialized' may mean, for example, readily found in nature or synthesized in a laboratory or chemical plant. In any event, a materialized composition presents a tangible reality. The terms 'to be compounded' or 'compounding' refer to the act of materialization of a composition, whether via extraction and assembly of ingredients or via synthetization and assembly of ingredients.

In the present invention, the term "stability" refers to a quantitative measurement of the evolution of a materialized composition in a base in terms either of, for example, solubility, colorimetry or olfactometry. This evolution may be measured at different time intervals. Such time intervals may vary depending on the application of the composition and base association. A stability value may be, in simple embodiments, either true or false, meaning the evolution measured for the composition in the base is less than a predetermined validity threshold. A stability value may be, in other embodiments, a numerical value representative of the evolution measured for the composition in the base. Such a value may correspond to, for example, a transparency or chromatic distance value. Such a value may also correspond to a variation in perceived psychophysical intensity or a change in the hedonics of the composition. Such a value may also correspond to the physical solubility of a fragrance within a base, resulting for example in the formation of separate phases or precipitate.

As such the methods and systems disclosed below may correspond to:
- methods and systems to predict the colorimetric stability of a fragrance in a base,
- methods and systems to predict the olfactive stability of a fragrance in a base and/or
- methods and systems to predict the solubility stability of a fragrance in a base.

Such stability criteria are, however, not limitative in assessing the scope of the present invention.

The embodiments disclosed below are presented in a general manner.

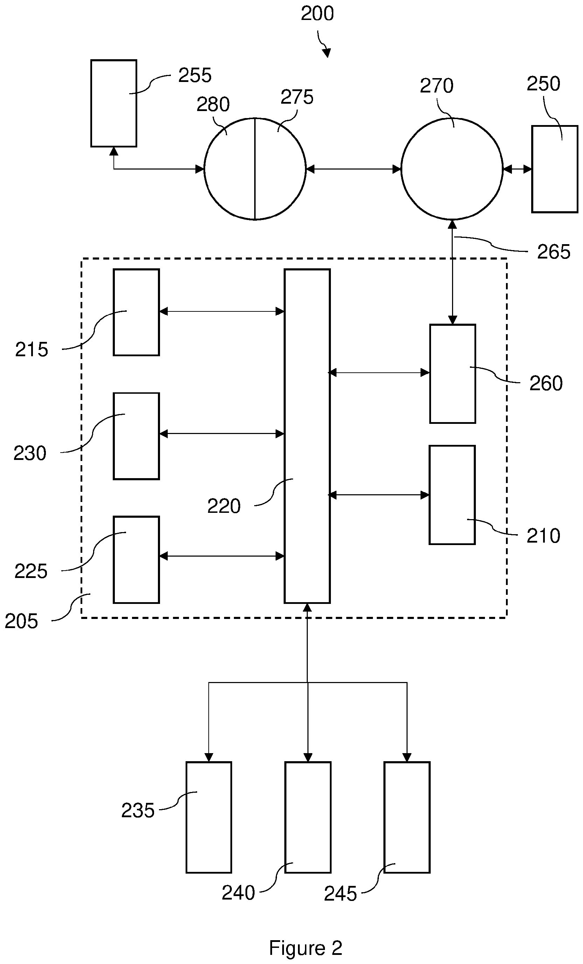

Figure 2 represents a block diagram that illustrates an example computer system with which an embodiment may be implemented. In the example of figure 2, a computer system 205 and instructions for implementing the disclosed technologies in hardware, software, or a combination of hardware and software, are represented schematically, for example as boxes and circles, at the same level of detail that is commonly used by persons of ordinary skill in the art to which this disclosure pertains for communicating about computer architecture and computer systems implementations.

The computer system 205 includes an input/output (IO) subsystem 220 which may include a bus and/or other communication mechanism(s) for communicating information and/or instructions between the components of the computer system 205 over electronic signal paths. The I/O subsystem 220 may include an I/O controller, a memory controller and at least one I/O port. The electronic signal paths are represented schematically in the drawings, for example as lines, unidirectional arrows, or bidirectional arrows.

At least one hardware processor 210 is coupled to the I/O subsystem 220 for processing information and instructions. Hardware processor 210 may include, for example, a general-purpose microprocessor or microcontroller and/or a special-purpose microprocessor such as an embedded system or a graphics processing unit (GPU) or a digital signal processor or ARM processor. Processor 210 may comprise an integrated arithmetic logic unit (ALU) or may be coupled to a separate ALU.

Computer system 205 includes one or more units of memory 225, such as a main memory, which is coupled to I/O subsystem 220 for electronically digitally storing data and instructions to be executed by processor 210. Memory 225 may include volatile memory such as various forms of random-access memory (RAM) or other dynamic storage device. Memory 225 also may be used for storing temporary variables or other intermediate information during execution of instructions to be executed by processor 210. Such instructions, when stored in non-transitory computer-readable storage media accessible to processor 210, can render computer system 205 into a special-purpose machine that is customized to perform the operations specified in the instructions.

Computer system 205 further includes non-volatile memory such as read only memory (ROM) 230 or other static storage device coupled to the I/O subsystem 220 for storing information and instructions for processor 210. The ROM 230 may include various forms of programmable ROM (PROM) such as erasable PROM (EPROM) or electrically erasable PROM (EEPROM). A unit of persistent storage 215 may include various forms of non-volatile RAM (NVRAM), such as FLASH memory, or solid-state storage, magnetic disk, or optical disk such as CD-ROM or DVD-ROM and may be coupled to I/O subsystem 220 for storing information and instructions. Storage 215 is an example of a non-transitory computer-readable medium that may be used to store instructions and data which when executed by the processor 210 cause performing computer-implemented methods to execute the techniques herein.

The instructions in memory 225, ROM 230 or storage 215 may comprise one or more sets of instructions that are organized as modules, methods, objects, functions, routines, or calls. The instructions may be organized as one or more computer programs, operating system services, or application programs including mobile apps. The instructions may comprise an operating system and/or system software; one or more libraries to support multimedia, programming or other functions; data protocol instructions or stacks to implement TCP/IP, HTTP or other communication protocols; file format processing instructions to parse or render files coded using HTML, XML, JPEG, MPEG or PNG; user interface instructions to render or interpret commands for a graphical user interface (GUI), command-line interface or text user interface; application software such as an office suite, internet access applications, design and manufacturing applications, graphics applications, audio applications, software engineering applications, educational applications, games or miscellaneous applications. The instructions may implement a web server, web application server or web client. The instructions may be organized as a presentation layer, application layer and data storage layer such as a relational database system using structured query language (SQL) or no SQL, an object store, a graph database, a flat file system or other data storage.

Computer system 205 may be coupled via I/O subsystem 220 to at least one output device 235. In one embodiment, output device 235 is a digital computer display. Examples of a display that may be used in various embodiments include a touch screen display or a light-emitting diode (LED) display or a liquid crystal display (LCD) or an e-paper display. Computer system 205 may include other type(s) of output devices 235, alternatively or in addition to a display device. Examples of other output devices 235 include printers, ticket printers, plotters, projectors, sound cards or video cards, speakers, buzzers or piezoelectric devices or other audible devices, lamps or LED or LCD indicators, haptic devices, actuators, or servos.

At least one input device 240 is coupled to I/O subsystem 220 for communicating signals, data, command selections or gestures to processor 210. Examples of input devices 240 include touch screens, microphones, still and video digital cameras, alphanumeric and other keys, keypads, keyboards, graphics tablets, image scanners, joysticks, clocks, switches, buttons, dials, slides.

Another type of input device is a control device 245, which may perform cursor control or other automated control functions such as navigation in a graphical interface on a display screen, alternatively or in addition to input functions. Control device 245 may be a touchpad, a mouse, a trackball, or cursor direction keys for communicating direction information and command selections to processor 210 and for controlling cursor movement on display 235. The input device may have at least two degrees of freedom in two axes, a first axis (e.g., x) and a second axis (e.g., y), that allows the device to specify positions in a plane. Another type of input device is a wired, wireless, or optical control device such as a joystick, wand, console, steering wheel, pedal, gearshift mechanism or other type of control device. An input device 240 may include a combination of multiple different input devices, such as a video camera and a depth sensor.

In another embodiment, computer system 205 may comprise an internet of things (IoT) device in which one or more of the output device 235, input device 240, and control device 245 are omitted. Or, in such an embodiment, the input device 240 may comprise one or more cameras, motion detectors, thermometers, microphones, seismic detectors, other sensors or detectors, measurement devices or encoders and the output device 235 may comprise a special-purpose display such as a single-line LED or LCD display, one or more indicators, a display panel, a meter, a valve, a solenoid, an actuator or a servo.

Computer system 205 may implement the techniques described herein using customized hard-wired logic, at least one ASIC or FPGA, firmware and/or program instructions or logic which when loaded and used or executed in combination with the computer system causes or programs the computer system to operate as a special-purpose machine. According to one embodiment, the techniques herein are performed by computer system 205 in response to processor 210 executing at least one sequence of at least one instruction contained in main memory 225. Such instructions may be read into main memory 225 from another storage medium, such as storage 215. Execution of the sequences of instructions contained in main memory 225 causes processor 210 to perform the process steps described herein. In alternative embodiments, hard-wired circuitry may be used in place of or in combination with software instructions.

The term "storage media" as used herein refers to any non-transitory media that store data and/or instructions that cause a machine to operation in a specific fashion. Such storage media may comprise non-volatile media and/or volatile media. Non-volatile media includes, for example, optical or magnetic disks, such as storage 215. Volatile media includes dynamic memory, such as memory 225. Common forms of storage media include, for example, a hard disk, solid state drive, flash drive, magnetic data storage medium, any optical or physical data storage medium, memory chip, or the like.

Storage media is distinct from but may be used in conjunction with transmission media. Transmission media participates in transferring information between storage media. For example, transmission media includes coaxial cables, copper wire and fiber optics, including the wires that comprise a bus of I/O subsystem 220. Transmission media can also take the form of acoustic or light waves, such as those generated during radiowave and infra-red data communications.

Various forms of media may be involved in carrying at least one sequence of at least one instruction to processor 210 for execution. For example, the instructions may initially be carried on a magnetic disk or solid-state drive of a remote computer. The remote computer can load the instructions into its dynamic memory and send the instructions over a communication link such as a fiber optic or coaxial cable or telephone line using a modem. A modem or router local to computer system 205 can receive the data on the communication link and convert the data to a format that can be read by computer system 205. For instance, a receiver such as a radio frequency antenna or an infrared detector can receive the data carried in a wireless or optical signal and appropriate circuitry can provide the data to I/O subsystem 220 such as place the data on a bus. I/O subsystem 220 carries the data to memory 225, from which processor 210 retrieves and executes the instructions. The instructions received by memory 225 may optionally be stored on storage 215 either before or after execution by processor 210.

Computer system 205 also includes a communication interface 260 coupled to bus 220. Communication interface 260 provides a two-way data communication coupling to network link(s) 265 that are directly or indirectly connected to at least one communication networks, such as a network 270 or a public or private cloud on the Internet. For example, communication interface 260 may be an Ethernet networking interface, integrated-services digital network (ISDN) card, cable modem, satellite modem, or a modem to provide a data communication connection to a corresponding type of communications line, for example an Ethernet cable or a metal cable of any kind or a fiber-optic line or a telephone line. Network 270 broadly represents a local area network (LAN), wide-area network (WAN), campus network, internetwork, or any combination thereof. Communication interface 260 may comprise a LAN card to provide a data communication connection to a compatible LAN, or a cellular radiotelephone interface that is wired to send or receive cellular data according to cellular radiotelephone wireless networking standards, or a satellite radio interface that is wired to send or receive digital data according to satellite wireless networking standards. In any such implementation, communication interface 260 sends and receives electrical, electromagnetic, or optical signals over signal paths that carry digital data streams representing various types of information.

Network link 265 typically provides electrical, electromagnetic, or optical data communication directly or through at least one network to other data devices, using, for example, satellite, cellular, Wi-Fi, or BLUETOOTH technology. For example, network link 265 may provide a connection through a network 270 to a host computer 250.

Furthermore, network link 265 may provide a connection through network 270 or to other computing devices via internetworking devices and/or computers that are operated by an Internet Service Provider (ISP) 275. ISP 275 provides data communication services through a world-wide packet data communication network represented as internet 280. A server computer 255 may be coupled to internet 280. Server 255 broadly represents any computer, data center, virtual machine, or virtual computing instance with or without a hypervisor, or computer executing a containerized program system such as DOCKER or KUBERNETES. Server 255 may represent an electronic digital service that is implemented using more than one computer or instance and that is accessed and used by transmitting web services requests, uniform resource locator (URL) strings with parameters in HTTP payloads, API calls, app services calls, or other service calls. Computer system 205 and server 255 may form elements of a distributed computing system that includes other computers, a processing cluster, server farm or other organization of computers that cooperate to perform tasks or execute applications or services. Server 255 may comprise one or more sets of instructions that are organized as modules, methods, objects, functions, routines, or calls. The instructions may be organized as one or more computer programs, operating system services, or application programs including mobile apps. The instructions may comprise an operating system and/or system software; one or more libraries to support multimedia, programming or other functions; data protocol instructions or stacks to implement TCP/IP, HTTP or other communication protocols; file format processing instructions to parse or render files coded using HTML, XML, JPEG, MPEG or PNG; user interface instructions to render or interpret commands for a graphical user interface (GUI), command-line interface or text user interface; application software such as an office suite, internet access applications, design and manufacturing applications, graphics applications, audio applications, software engineering applications, educational applications, games or miscellaneous applications. Server 255 may comprise a web application server that hosts a presentation layer, application layer and data storage layer such as a relational database system using structured query language (SQL) or no SQL, an object store, a graph database, a flat file system or other data storage.

Computer system 205 can send messages and receive data and instructions, including program code, through the network(s), network link 265 and communication interface 260. In the Internet example, a server 255 might transmit a requested code for an application program through Internet 280, ISP 275, local network 270 and communication interface 260. The received code may be executed by processor 210 as it is received, and/or stored in storage 215, or other non-volatile storage for later execution.

The execution of instructions as described in this section may implement a process in the form of an instance of a computer program that is being executed and consisting of program code and its current activity. Depending on the operating system (OS), a process may be made up of multiple threads of execution that execute instructions concurrently. In this context, a computer program is a passive collection of instructions, while a process may be the actual execution of those instructions. Several processes may be associated with the same program; for example, opening up several instances of the same program often means more than one process is being executed. Multitasking may be implemented to allow multiple processes to share processor 210. While each processor 210 or core of the processor executes a single task at a time, computer system 205 may be programmed to implement multitasking to allow each processor to switch between tasks that are being executed without having to wait for each task to finish. In an embodiment, switches may be performed when tasks perform input/output operations, when a task indicates that it can be switched, or on hardware interrupts. Time-sharing may be implemented to allow fast response for interactive user applications by rapidly performing context switches to provide the appearance of concurrent execution of multiple processes simultaneously. In an embodiment, for security and reliability, an operating system may prevent direct communication between independent processes, providing strictly mediated and controlled inter-process communication functionality.

Figure 2 further shows a system 200 for predicting a stability value for a determined fragrance in a determined fragrance base, characterized in that it comprises:
- one or more computer systems 205 comprising one or more hardware processors 210 and storage 215 media; and
- instructions stored in the storage media and which, when executed by the computing system, cause the computing system to perform:
   - a step of inputting, upon a computer interface, at least two fragrance ingredient digital identifiers, representative of material fragrance ingredients, the resulting input corresponding to a composition of fragrance ingredients,
   - a step of inputting, upon a computer interface, a fragrance base digital identifier, representative of a material fragrance base,
   - a step of determination, by a computing device, of a stability value for the input composition in the input fragrance base, as a function of the input composition and the input fragrance base and
   - a step of providing, upon a computer interface, a value representative of the determined stability value.

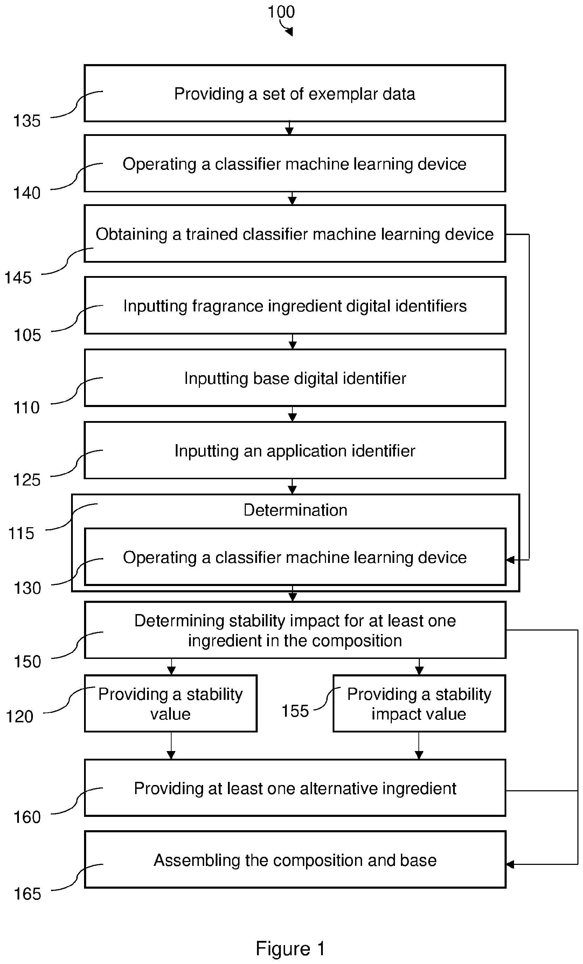

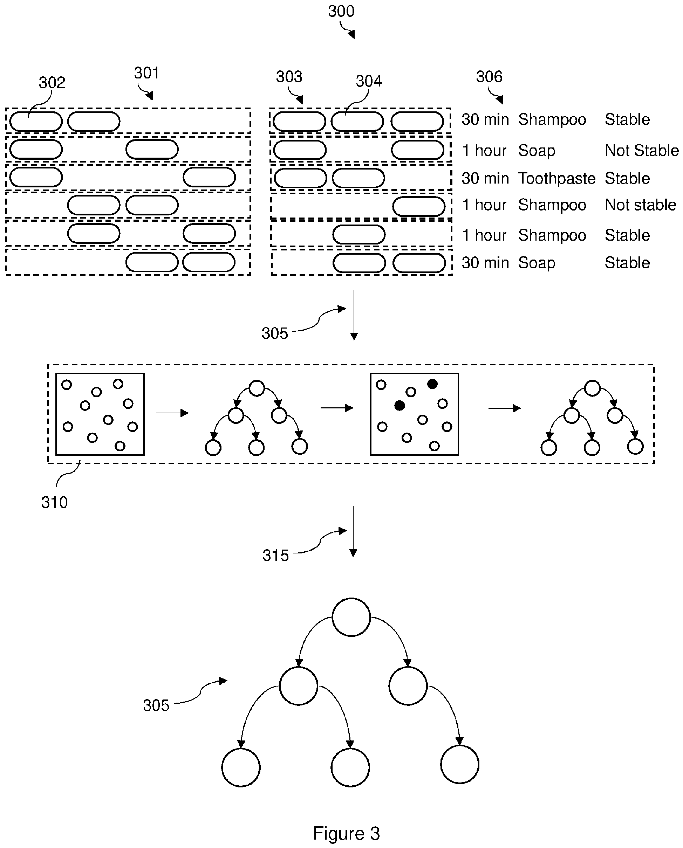

Particular embodiments of the steps above are disclosed in regard of figure 1 and 3.

Figure 1 shows, schematically, a particular succession of steps of the method 100 object of the present invention. This method 100 for predicting a stability value for a determined fragrance in a determined fragrance base, comprises:
- a step 105 of inputting, upon a computer interface, at least two fragrance ingredient digital identifiers, representative of material fragrance ingredients, the resulting input corresponding to a composition of fragrance ingredients,
- a step 110 of inputting, upon a computer interface, a fragrance base digital identifier, representative of a material fragrance base,
- a step 115 of determination, by a computing device, of a stability value for the input composition in the input fragrance base, as a function of the input composition and the input fragrance base and
- a step 120 of providing, upon a computer interface, a value representative of the determined stability value.

The step 105 of inputting is performed, for example, manually or automatically upon a computer interface, such as a graphical user interface ("GUI") associated to an I/O subsystem 220 coupled with an input device 240, such as shown in figure 2. During this step 105 of inputting, fragrance ingredient digital identifiers may be selected to form a composition. Such fragrance ingredient digital identifiers may be represented as buttons, items in a list or items that can be searched via a search engine. A user may add any number of ingredients, represented by the corresponding digital identifiers. This step 105 correspond to a potential or intended composition to be assembled and associated to a particular fragrance base.

In particular embodiments, relative or absolute quantities of said fragrance ingredient digital identifiers, within the composition, may be input.

At the outset of this step 105 of inputting, a composition is defined.

The step 110 of inputting is performed, for example, manually or automatically upon a computer interface, such as a graphical user interface ("GUI") associated to an I/O subsystem 220 coupled with an input device 240, such as shown in figure 2. During this step 110 of inputting, a base digital identifier may be selected. Such a base digital identifier may be represented as a button, an item in a list or an item that can be searched via a search engine. A user may add any number of bases, represented by the corresponding digital identifiers. This step 110 correspond to a potential or intended base to be associated with the input composition.

In particular embodiments, the step 110 of inputting is performed by selecting base constituents, such as molecules or compounds constitutive of the base, represented by corresponding base constituent digital identifiers, the selection of base constituent digital identifiers forming a base composition.

In particular embodiments, such as the one represented in figure 1, the method 100 object of the present invention comprises a step 125 of inputting, upon a computer interface, an fragrance application digital identifier, representative of the use the input fragrance and the input base.

The step 125 of inputting is performed, for example, manually or automatically upon a computer interface, such as a graphical user interface ("GUI") associated to an I/O subsystem 220 coupled with an input device 240, such as shown in figure 2. During this step 125 of inputting, an application identifier may be selected. Such an application digital identifier may be represented as a button, an item in a list or an item that can be searched via a search engine. A user may add any number of applications, represented by the corresponding digital identifiers. This step 110 correspond to a potential or intended use to be associated with at least one couple formed of a composition digital identifier and a base digital identifier.

The application digital identifier may correspond to an application or to a class of applications of any degree of abstraction resulting from a process of taxonomy.

The step 115 of determination may be performed, for example, by a computer software represented by instructions stored in a storage 215 media and executed by hardware processors 210 of a computer system 205. During this step 115 of determination, two main types of algorithms may be used:
- the first type corresponds to expert system modelling, in which a deterministic (and programmed) equation or system of equation links the presence and/or quantity of fragrance ingredients and the base to a stability value and
- the second type corresponds to the implementation of a machine learning algorithm on the basis of known data to infer, or predict, the stability of the combination of a composition and a fragrance base.

In the first type, the presence of a particular ingredient above a determined threshold may automatically set a value of stability to value corresponding to the absence of stability, for example. While such approaches are possible, the combinatory possibilities in large fragrance ingredient palettes, combined with large fragrance based palettes, requires significant manpower.

In the second type, supervised machine learning algorithms may be implemented. In such algorithms, training data corresponding to sets of compositions and fragrance based, associated with values representative of the stability of said compositions and fragrances, are fed to a training algorithm to produce a trained machine learning device.

In particular embodiments, such as the one represented in figure 1, the method 100 object of the present invention comprises the steps of:
- providing 135 to a classifier machine learning device a set of exemplar data, comprising:
   - composition digital identifiers, each composition digital identifier being associated with fragrance ingredient digital identifiers,
   - for at least part of the exemplar composition digital identifiers, a fragrance base digital identifier, and
   - for at least one couple formed of the exemplar composition and base digital identifiers, a value representative of stability of the exemplar composition,
- operating 140 the classifier machine learning device based upon the set of exemplar data and
- obtaining 145 the trained classifier machine learning device.

The step 135 of providing may be performed by using the I/O subsystem 220 of a computing system 205, coupled with storage 215 media or with an API feeding the input exemplar data.

A particular embodiment of the exemplar data is represented in figure 3. This schematic representation shows that such an input may comprise:
- compositions 301, each composition being represented as a sum of ingredients 302, the ingredients varying in number and nature between the different compositions, such ingredients being represented by digital identifiers, such as alphanumerical labels, representative of a reference in a database of ingredient digital representations,
- fragrance bases 303, each fragrance base being represented as a sum of base constituents 304, the base constituents varying in number and nature between the different fragrance bases, such base constituents being represented by digital identifiers, such as alphanumerical labels, representative of a reference in a database of base constituents digital representations - alternatively, fragrance bases 303 may be considered as whole entities (that is, not decomposed to the constituent level), represented by digital identifiers representative of a digital representation of said fragrance bases and
- attributes 306, linked to the association of a composition and a fragrance base, such as duration since association to stability measurement, or a related application.

In particular embodiments, the exemplar data further comprises, for at least one couple formed of exemplar composition and base digital identifiers, a value representative of duration from assembly of said couple.

In particular embodiments, the exemplar data further comprises for at least one couple formed of exemplar composition and base digital identifiers, a value representative of the concentration of the composition within the base of said couple.

In particular embodiments, the exemplar data further comprises for at least one couple formed of exemplar composition and base digital identifiers, a value representative of an application for said couple.

Figure 3 represents the step 135 of providing as an arrow 305 feeding this input into a machine learning device to be trained.

The step 140 of operating the machine learning device is performed, for example, by one or more hardware processors 210 configured to execute a set of instructions representative of the machine learning device. Such a machine learning device corresponds, for example, to a gradient boosting decision tree device, referenced 305 in figure 3. This step 140 of operating is represented by reference 310 in figure 3. Preferentially, gradient boosting decision trees are built sequentially, so that each new tree minimizes classification loss.

In a particular example, a dataset formed of over 100 000 pass/fail observations (meaning "stable/not stable") for over 30 000 formulas and containing the following explanatory variables may be used:
- formula upn defines the identifier of the chemical formula of the perfume,
- fragrance dosage measures the concentration of the formula inside the tested product (%),
- segment name defines at a high level the usage of the product: Body and Personal Care, Home and Fabric Care, Fine fragrance,
- subsegment name defines the usage of the product in a more specific manner than segment name: 13 attributes,
- application name defines the usage of the product in a more specific manner than subsegment name - it corresponds to the leaf of the tree structure defined by segment name, subsegment name, application name: 158 attributes and
- matcode defines that base that was used in the stability test.

The formula identifier, upn, can be associated with a sparse matrix called composition matrix storing the weighted partition of the used ingredients, where the weighted sum for each formula adds up to 1. The composition matrix has approximately 1 million formulas (rows) and 6 000 ingredients (columns) where the weight corresponds to the quantity of the given ingredient (column) inside the given formula (row). This allows the replacement of a formula identifier by its corresponding ingredient composition.

In particular embodiments, a grid search algorithm may be used to select the best set of parameters for and therefore maximize the results for each stability prediction:
- encapsulation name: present or not depending on the use of an encapsulation technology to sequester a fragrance and/or
- olfactive list: a set of olfactive descriptors, such as labels describing the perceived description of a smell of a fragrance, used as features.

An olfactive descriptor, or olfactive description, describes how something smells. In other words, the olfactive descriptor corresponds to the organoleptic property imparted by a perfuming composition, or composition of fragrance ingredients, or a perfuming ingredient, or ingredient. Such an olfactive descriptor may correspond to, items that a particular fragrance smells similar to, such as "Citrus", "Aromatic", "Red fruit", Rose", for example. Several olfactive descriptor may be used for one perfuming composition or one perfuming ingredient.

A taste descriptor, or taste description, is similar in scope, only for taste instead of smell.

To maximize the gain of information brought by the use of target encoding, it is possible to compute, for stability, the features having the most importance. A feature is considered as the most important, if the accuracy score difference between adding and deleting the feature is maximized.

The step 145 of obtaining the trained machine learning device is performed, for example, by one or more hardware processors 210 configured to execute a set of instructions representative of a storage, in a storage 215 media, of the result of the step 140 of operating. This step 145 of obtaining is represented by reference 315 in figure 3.

This trained machine learning device, or model, is configured to receive the input composition, fragrance base and optionally application and predict, as an output, the stability of this association of inputs. This corresponds to the step of 130 of operating the machine learning device or model.

In particular embodiments, such as the one represented in figure 1, the step 115 of determination comprises a step 130 of operating a trained classifier machine learning device, in which input composition and the input fragrance base are used as input of the classifier machine learning model and in which the output is a stability class identifier, representative of a stability of the input composition in the input fragrance base.

The step 120 of providing may be performed, for example, upon a computer interface, such as a graphical user interface ("GUI") associated to an I/O subsystem 220 coupled with an output device 235, such as shown in figure 2. During this step 120 of providing, the predicted stability may be represented as a numerical or alphabetical value.

In particular embodiments, such as the one shown in figure 1, the method 100 object of the present invention comprises, downstream of the step 130 of operating the trained gradient boosting decision tree device, a step 150 of determining, by a computing device, a numerical value representative of a stability impact for at least one input fragrance ingredient digital identifier and a step 155 of providing, upon a computer interface, the determined stability impact numerical value.

The step 150 of determining is performed, for example, for example, by one or more hardware processors 210 configured to execute a set of instructions representing a software.

To determine which set of ingredients are the most influential for stability, it is important to remember stability scores are influenced by the application and the base. Therefore ingredient recommendations are supplied for an application-base combination. Recognized as the state of the art method in Machine Learning explainability, the Shapley Additive Explanations (SHAP) was published in 2017 by Lundberg and Lee. SHAP assigns each feature of the input dataset, an importance value for a particular prediction. Selecting the most influential ingredients is not a trivial task considering that the ingredients having a positive or a negative relationship between the SHAP importance scores and the ingredient dosages need to be treated separately. Linear relationships may be determined via Spearman correlations and enable the separation of positives from negatives relationships.

This allows to find the ingredients giving the most positive influence for stability, which can be associated with boundary dosage given by a minimum and/or a maximum quantity for the specific ingredient. The minimum quantity for a particular ingredient should indicate the starting quantity where the ingredient becomes an enhancer for stability. Going above the minimum quantity may increase the stability of the given fragrance until the maximum quantity is reached, defined as the break point. Adding more quantity than indicated by the maximum quantity result in a slow increase or a saturation or a decrease in the influence of the ingredient. Ingredients having the most negative influence also gives important additional information to perfumers. Adding the negative ingredients in high quantities could drastically decrease stability. For this reason, it is possible to compute only the maximum dosage, which determines the limit for the quantity a perfumer can add before the ingredient contribution become negative and therefore become a disruptor for the stability.

Considering the positive or negative relationships, the final importance score per ingredient is computed by taking the median over the positive or negative SHAP scores for each ingredient owing more than 50 positive or negative samples. In such manner, ingredients contributing the most positively or negatively are the ones having the highest or lowest median score. However, it is expected that the most influential ingredients to be the ones maximizing both the median SHAP values together with the correlation score, in order to have a discernible separation between the positive and negative contributions while maximizing this contribution.

With the aim to maximize both parameters, it is possible to normalize between 0 and 1 the correlation and the SHAP scores in such a way that both scores have equivalent weights. The final impact score, assessing the influence of an ingredient is finally obtained by summing these normalized scores, 2 being the maximum and 0 the minimum. Once the most influential ingredients (also called actionable ingredients) have been computed, the computation of the quantity boundaries require the detection and the deletion of the outlier data samples in order to maximize their reliability.

In particular embodiments, such as the one shown in figure 1, the method 100 object of the present invention comprises, downstream of the step 150 of determining a numerical value representative of a stability impact, a step 160 of providing at least one alternative fragrance ingredient digital identifier for at least one fragrance ingredient digital identifier input to form the composition as function of the numerical value representative of a stability impacts of the said input and of said alternative fragrance ingredient digital identifiers.

The step 160 of providing is performed, for example, for example, by one or more hardware processors 210 configured to execute a set of instructions representing a software. During this step 160 of providing, other alternative fragrance ingredient digital identifiers that were not initially input or variations of concentrations of fragrance ingredient digital identifiers are used in lieu of at least one initially input fragrance ingredient digital identifier and the stability performance of the composition-base combination is reassessed. If the performance increases, the alternative fragrance ingredient digital identifier or the alternative concentration is provided, for example, to a user upon a GUI.

In particular embodiments, such as the one shown in figure 1, the step 160 of providing at least one alternative fragrance ingredient digital identifier is configured to further provide at least one value representative of a concentration of at least one said alternative fragrance ingredient digital identifier.

With the intention to determine the quantity ranges and boundaries, it is possible to approximate, for each actionable ingredient, the data points appearing in the SHAP versus the quantity plot through piecewise linear functions. The optimal number of linear functions may be set to three, giving the major trends while enabling a straightforward analysis to determine the quantity boundaries.

It is possible that graphing the ingredient quantity usage shows a decreasing number of usages as the quantity of the ingredient is increasing. Passing a certain quantity, the density of data points may become small enough to say that the approximation line is no longer reliable compared to its reliability. In certain cases, the density of points can be so low that a single data point can completely change the slope of the linear function. To be able to counter this effect, it is possible to develop a method of finding the threshold quantity from which approximation line can be considered as not reliable anymore. We separate the quantity range in buckets of the same size in order to count the number of data points contained in each bucket. A threshold on the number of data points per bucket is defined as:
thresholdbucket = 1 /|buckets|·2 ∑b∈buckets ∑x∈b 1(xquantity>0) where x represents a data point contained in bucket b and 1(xquantity>0) represents the indicator function with the condition that the dosage of data point x should be strictly greater than 0 and the threshold on the quantity is defined as the maximum quantity of the last bucket fulfilling the condition defined above.

The three piecewise linear functions are useful to assess the boundary quantities which will differ depending on the positive or negative contribution of the chosen ingredient. The minimum quantity can be computed by projecting the 0 SHAP score on the approximation line. The choice of selecting piecewise linear functions is needed to compute the maximum quantity for positive contributors. The break points may be referred to as the two extremities points of the gradient line, as well as the points where the slope changes, giving a total of four break points for most ingredients. The maximum quantity can be obtained by selecting the break point through an analysis of the three slopes and identically through an analysis of the three gradients. Let grad 0, grad 1, grad 2 be the gradient computed for the three linear functions respectively, and break 0, break 1, break 2, break 3 be the break points. The pseudo code below shows the analyses done to obtain the maximum quantity for positive contributor ingredients:

```
       Condition 1: the gradients decrease
       if grad_0 > grad_1 > grad_2 and grad_1 > 0
       return break_2
       # Condition 2: the gradients increase
       elif grad_2 > grad_1 > grad_0 and grad_1 > 0
       return break_3
       # Condition 3: the last gradient is greater than the second one
       elif grad_2 > grad_1
       return break_1
       # Condition 4: the first gradient is greater than the first one
       elif grad_1 > grad_0
       return break_2
       # Others
       else
       return break_1
```

For the disruptor ingredients, it is possible to apply the outlier deletion process and the maximum quantity can be obtained by projecting the zero SHAP score on the approximation line. Since the linear correlation between the SHAP score and the quantity is negative, the 0 SHAP score indicates the maximum quantity of that ingredient one can add, before the contribution becomes negative with a negative SHAP score.

In particular embodiments, such as the one shown in figure 1, the step 160 of providing at least one alternative fragrance ingredient digital identifier is configured to further provide a minimum and/or a maximum value representative of a concentration of at least one said alternative fragrance ingredient digital identifier.

In particular embodiments, such as the one shown in figure 1, the method 100 object of the present invention comprises a step 165 of assembling the input composition in the input fragrance base.

This step 165 of assembling is configured to materialize the composition. Such a step 165 of assembling may be performed in a variety of ways, such as in a laboratory or a chemical plant for example.

Figure 2 shows, schematically, a particular embodiment of the system 200 object of the present invention. This system 200 for predicting a stability value for a determined fragrance in a determined fragrance base comprises:
- one or more computer systems 205 comprising one or more hardware processors 210 and storage 215 media; and
- instructions stored in the storage media and which, when executed by the computing system, cause the computing system to perform:
- a step of inputting, upon a computer interface, at least two fragrance ingredient digital identifiers, representative of material fragrance ingredients, the resulting input corresponding to a composition of fragrance ingredients,
- a step of inputting, upon a computer interface, a fragrance base digital identifier, representative of a material fragrance base,
- a step of determination, by a computing device, of a stability value for the input composition in the input fragrance base, as a function of the input composition and the input fragrance base and
- a step of providing, upon a computer interface, a value representative of the determined stability value.

A description of optional embodiments for the corresponding means and steps is disclosed above.

## Claims

1. Method (100) for predicting a stability value for a determined fragrance in a determined fragrance base, **characterized in that** it comprises:
- a step (105) of inputting, upon a computer interface, at least two fragrance ingredient digital identifiers, representative of material fragrance ingredients, the resulting input corresponding to a composition of fragrance ingredients,
- a step (110) of inputting, upon a computer interface, a fragrance base digital identifier, representative of a material fragrance base,
- a step (115) of determination, by a computing device, of a stability value for the input composition in the input fragrance base, as a function of the input composition and the input fragrance base and
- a step (120) of providing, upon a computer interface, a value representative of the determined stability value.

2. Method (100) according to claim 1, which comprises a step (125) of inputting, upon a computer interface, an fragrance application digital identifier, representative of the use the input fragrance and the input base.

3. Method (100) according to any one of claims 1 or 2, in which the step (115) of determination comprises a step (130) of operating a trained classifier machine learning device, in which input composition and the input fragrance base are used as input of the classifier machine learning model and in which the output is a stability class identifier, representative of a stability of the input composition in the input fragrance base.

4. Method (100) according to claim 3, which comprises, upstream of the step (130) of operating a trained classifier machine learning device, the steps of:
- providing (135) to a classifier machine learning device a set of exemplar data, comprising:
- composition digital identifiers, each composition digital identifier being associated with fragrance ingredient digital identifiers,
- for at least part of the exemplar composition digital identifiers, a fragrance base digital identifier, and
- for at least one couple formed of the exemplar composition and base digital identifiers, a value representative of stability of the exemplar composition,
- operating (140) the classifier machine learning device based upon the set of exemplar data and
- obtaining (145) the trained classifier machine learning device.

5. Method (100) according to claim 4, in which the exemplar data further comprises, for at least one couple formed of exemplar composition and base digital identifiers, a value representative of duration from assembly of said couple.

6. Method (100) according to any one of claims 4 or 5, in which the exemplar data further comprises for at least one couple formed of exemplar composition and base digital identifiers, a value representative of the concentration of the composition within the base of said couple.

7. Method (100) according to any one of claims 4 to 6, in which the exemplar data further comprises for at least one couple formed of exemplar composition and base digital identifiers, a value representative of an application for said couple.

8. Method (100) according to any one of claims 3 to 7, in which the trained classifier machine learning model is a classifier gradient boosting decision tree device.

9. Method (100) according to any one of claims 3 to 8, comprising, downstream of the step (130) of operating the trained gradient boosting decision tree device, a step (150) of determining, by a computing device, a numerical value representative of a stability impact for at least one input fragrance ingredient digital identifier and a step (155) of providing, upon a computer interface, the determined stability impact numerical value.

10. Method (100) according to claim 9, comprising, downstream of the step (150) of determining a numerical value representative of a stability impact, a step (160) of providing at least one alternative fragrance ingredient digital identifier for at least one fragrance ingredient digital identifier input to form the composition as function of the numerical value representative of a stability impacts of the said input and of said alternative fragrance ingredient digital identifiers.

11. Method (100) according to claim 10, in which the step (160) of providing at least one alternative fragrance ingredient digital identifier is configured to further provide at least one value representative of a concentration of at least one said alternative fragrance ingredient digital identifier.

12. Method (100) according to any one of claims 9 or 10, in which the step (160) of providing at least one alternative fragrance ingredient digital identifier is configured to further provide a minimum and/or a maximum value representative of a concentration of at least one said alternative fragrance ingredient digital identifier.

13. Method (100) according to any one of claims 1 to 12, comprising a step (165) of assembling the input composition in the input fragrance base.

14. System (200) for predicting a stability value for a determined fragrance in a determined fragrance base, **characterized in that** it comprises:
- one or more computer systems (205) comprising one or more hardware processors (210) and storage (215) media; and
- instructions stored in the storage media and which, when executed by the computing system, cause the computing system to perform:
- a step of inputting, upon a computer interface, at least two fragrance ingredient digital identifiers, representative of material fragrance ingredients, the resulting input corresponding to a composition of fragrance ingredients,
- a step of inputting, upon a computer interface, a fragrance base digital identifier, representative of a material fragrance base,
- a step of determination, by a computing device, of a stability value for the input composition in the input fragrance base, as a function of the input composition and the input fragrance base and
- a step of providing, upon a computer interface, a value representative of the determined stability value.
